# EUROPEAN PATENT APPLICATION

(11) **EP 3 415 079 A1**
(43) Date of publication of application: **19.12.2018**
(21) Application number: 17175829.5
(22) Date of filing: 13.06.2017
(51) Int. Cl.: A61B 5/00

(54) **AN APPARATUS FOR RECEIVING AN INPUT INDICATIVE OF ONE OR MORE SYMPTOMS OF A SUBJECT AND A METHOD OF OPERATING THE SAME**

(71) Applicant: Koninklijke Philips N.V., 5656 AE Eindhoven (NL)
(72) Inventor: SARTOR, Francesco, 5656 AE Eindhoven (NL); HOLMES, Roger, 5656 AE Eindhoven (NL); SHRUBSOLE, Paul Anthony, 5656 AE Eindhoven (NL); WAANDERS, Leonie Francelle, 5656 AE Eindhoven (NL)
(74) Representative: de Haan, Poul Erik

(57) **Abstract**

There is provided an apparatus comprising a control unit and a method (200) of operating the apparatus for receiving an input indicative of one or more symptoms of a subject. Data is acquired on the subject from at least one sensor (202). An input is received from a user interface from the subject indicative of one or more symptoms of the subject (204). An onset time of the one or more symptoms is determined (206) based on the data acquired on the subject from the at least one sensor.

## Description

### Technical Field of the Invention

The invention relates to the field of healthcare and, in particular, to an apparatus for receiving an input indicative of one or more symptoms of a subject and a method of operating the apparatus.

### Background to the Invention

The accurate reporting of symptoms such as chest pain, breathlessness, dizziness, among others, experienced by a subject can be provide valuable information for medical personnel (for example, physicians) monitoring the subject. For example, in some cases, it is useful for subjects to precisely track how they feel when an event occurs. This can be helpful in the case of patients wearing a monitoring device, such as heart patients wearing a heart rate monitor or other type of monitoring tool.

The exact localisation in time of these symptoms provides key information for medical personnel, particularly when the symptoms are contextualised with physiological parameters (such as heart rate, respiration, blood pressure, and the like), behavioural parameters (for example, activities performed such as walking, sitting watching television, eating, and so on), and environmental parameters (such as being indoors or outdoors, a temperature of the environment, a humidity in the environment, and so on). For example, if a heart patient wearing a heart rate monitor suffers ischemia and concomitantly the patient experiences chest pain, the synchronisation of the two events can be useful in providing a better diagnosis. However, it is not possible to accurately determine the exact localisation of symptoms in time using current techniques.

For example, EP 2479692 discloses a method in which a time indication is provided for inputs. More specifically, EP 2479692 discloses receiving an input identifying a mood of a person and an input identifying an activity of the person coinciding with the mood along with a time indication for the inputs, which can be a timestamp. However, the timestamp is simply generated based on the time at which the inputs are received. As such, the timestamp does not accurately reflect the time at which a symptom actually started. On the contrary, there will inevitably be a time delay between the subject logging a symptom and the subject first experiencing the symptom, at least due to the time it takes for the subject to log the symptom. Currently, this manner of logging symptoms with a timestamp that has an inherited time lag is the only technique available for the temporal localisation of a received input indicative of symptoms experienced by a subject.

There is thus a need for an improved apparatus and method for receiving an input indicative of one or more symptoms of a subject.

### Summary of the Invention

As noted above, the limitation with existing approaches is that it is not possible to accurately localise in time the beginning of a symptom experienced by a subject for which an input is received. It would thus be valuable to have an improved apparatus and method for receiving an input indicative of one or more symptoms of a subject, which overcomes the existing problem.

Therefore, according to a first aspect of the invention, there is provided a method of operating an apparatus comprising a control unit for receiving an input indicative of one or more symptoms of a subject. The method comprises acquiring from at least one sensor data on the subject, receiving from a user interface an input from the subject indicative of one or more symptoms of the subject, and determining an onset time of the one or more symptoms based on the data acquired on the subject from the at least one sensor.

In some embodiments, the at least one sensor may comprise at least one physiological characteristic sensor and the data acquired on the subject may comprise physiological characteristic data on the subject.

In some embodiments, determining an onset time may comprise comparing the physiological characteristic data acquired prior to receipt of the input to reference physiological characteristic data stored in a memory, identifying a deviation between the physiological characteristic data acquired prior to receipt of the input and the reference physiological characteristic data, and determining the onset time of the one or more symptoms as the time at which the deviation started. The reference physiological characteristic data is indicative of a baseline for the physiological characteristic data.

In some embodiments, the at least one sensor may comprise at least one motion sensor, the data acquired on the subject may comprise motion data on the subject, and the method may further comprise, prior to comparing, identifying from the acquired motion data an activity in which the subject is engaging at the determined onset time. In these embodiments, comparing may comprise comparing the physiological characteristic data acquired prior to the determined onset time to the reference physiological characteristic data that is stored in the memory for the same activity as the identified activity. In some embodiments, the same activity may comprise any one or more of the same type of activity and the same intensity level of activity.

In some embodiments, the at least one sensor may comprise at least one motion sensor and the data acquired on the subject comprises motion data on the subject. In some embodiments, determining an onset time may comprise identifying, from the motion data acquired on the subject prior to receiving the input, a motion associated with the subject inputting the one or more symptoms at the user interface, and determining the onset time of the one or more symptoms as the time at which the identified motion associated with the subject inputting the one or more symptoms started.

In some embodiments, the method may further comprise comparing the physiological characteristic data acquired prior to the determined onset time to reference physiological characteristic data stored in a memory. The reference physiological characteristic data is indicative of a baseline for the physiological characteristic data. In these embodiments, the method may also further comprise determining whether a deviation between the physiological characteristic data acquired prior to the determined onset time and the reference physiological characteristic data occurred. Where a deviation exceeding a threshold deviation occurred, the determined onset time of the one or more symptoms may be changed to the time at which the deviation exceeding the threshold deviation occurred. Where no deviation occurred or a deviation less than the threshold deviation occurred, the determined onset time of the one or more symptoms may be maintained as the time at which the identified motion associated with the subject inputting the one or more symptoms started.

In some embodiments, the method may further comprise controlling a memory to store the one or more symptoms with the onset time of the one or more symptoms. In some embodiments, the method may further comprise outputting the one or more symptoms with the onset time of the one or more symptoms.

In some embodiments, the method may further comprise correlating the one or more symptoms to one or more physiological characteristics acquired at the onset time of the one or more symptoms, one or more behavioural parameters acquired at the onset time of the one or more symptoms, and/or one or more environmental parameters acquired at the onset time of the one or more symptoms. In some embodiments, the method may further comprise controlling a memory to store the correlation. In some embodiments, the method may further comprise outputting the correlation for use in diagnosis.

In some embodiments, the one or more symptoms may comprise any one or more of: pain in a part of the body of the subject, breathlessness of the subject, and dizziness of the subject. In some embodiments, the physiological characteristic data may comprise any one or more of: a heart rate data of the subject, a blood pressure data of the subject, and a respiratory rate data of the subject.

According to a second aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to a third aspect of the invention, there is provided an apparatus for receiving an input indicative of one or more symptoms of a subject. The apparatus comprises a control unit configured to acquire from at least one sensor data on the subject, receive from a user interface an input from the subject indicative of one or more symptoms of the subject, and determine an onset time of the one or more symptoms based on the data acquired on the subject from the at least one sensor.

In some embodiments, the apparatus may comprise one or more of the at least one sensors and/or the user interface.

According to the aspects and embodiments described above, the limitations of existing techniques are addressed. In particular, according to the above-described aspects and embodiments, it is possible to provide a more accurate temporal determination of a symptom occurring for which an input is received. As a more accurate indication can be provided of the instant in time at which the symptom actually started, it is possible to provide more precise and thus more valuable information about the subject. In this way, the information provided can be used to arrive at an improved diagnosis.

There is thus provided an improved apparatus and method for receiving an input indicative of one or more symptoms of a subject, which overcomes the problems associated with existing techniques.

### Brief Description of the Drawings

For a better understanding of the invention, and to show more clearly how it may be carried into effect, reference will now be made, by way of example only, to the accompanying drawings, in which:
Figure 1 is a block diagram of an apparatus according to an embodiment; and
Figure 2 is a flow chart illustrating a method according to an embodiment;
Figure 3 is a flow chart illustrating a method according to an example embodiment; and
Figure 4 is a flow chart illustrating a method according to an example embodiment.

### Detailed Description of the Preferred Embodiments

As noted above, the invention provides an improved apparatus and method for receiving an input indicative of one or more symptoms of a subject, which overcomes existing problems.

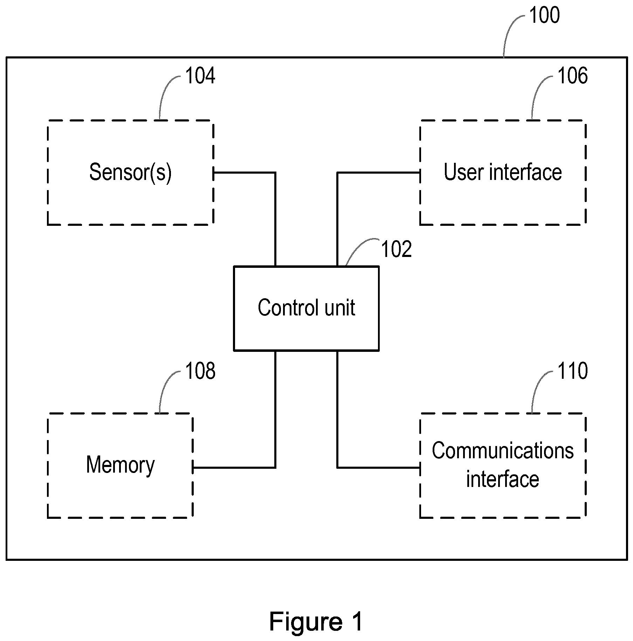

**Figure 1** illustrates an example of an apparatus 100 for receiving an input indicative of one or more symptoms of a subject according to an embodiment. The apparatus 100 can, for example, be a wearable device (for example, a smart watch, a monitoring device, or any other wearable device), a portable electronic device (for example, a smart device such as a tablet device, a smart phone, or any other portable electronic device), or any other type of device, or a processing resource within the cloud (for example, on a server in the cloud), or any other processing resource.

With reference to Figure 1, the apparatus 100 comprises a control unit 102 that controls the operation of the apparatus 100 and that can implement the method described herein. The control unit 102 can comprise one or more processors, processing units, multi-core processors or modules that are configured or programmed to control the apparatus 100 in the manner described herein. In particular implementations, the control unit 102 can comprise a plurality of software and/or hardware modules that are each configured to perform, or are for performing, individual or multiple steps of the method according to embodiments of the invention.

Briefly, the control unit 102 of the apparatus 100 is configured to acquire data on a subject from at least one sensor 104, receive from a user interface 106 an input from the subject indicative of one or more symptoms of the subject, and determine an onset time of the one or more symptoms based on the data acquired on the subject from the at least one sensor 104. In any of the embodiments disclosed herein, the one or more symptoms can comprise any one or more of pain in a part of the body (such as the chest, or any other part of the body) of the subject, breathlessness of the subject, dizziness of the subject, or any other symptom, or any combination of symptoms.

As illustrated in Figure 1, according to some embodiments, the apparatus 100 itself can comprise one or more of the at least one sensors 104 that are configured to acquire data on a subject. Alternatively or in addition, one or more of the at least one sensors 104 may be external to (i.e. separate to or remote from) the apparatus 100. For example, one or more of the at least one sensors 104 may be part of, or embedded in, another device. In some embodiments, any one or more of a wearable device (such as a smart watch, a monitoring device, or any other wearable device) configured to be worn by the subject, a portable electronic device (for example, a smart device such as a tablet device, a smart phone, or any other portable electronic device), and any other type of device may comprise one or more of the at least one sensors 104. Alternatively or in addition, one or more of the at least one sensors 104 may be provided in a predetermined location at which (or an environment in which) the subject is to be present when providing an input indicative of one or more symptoms.

According to some embodiments, the at least one sensor 104 can comprise at least one physiological characteristic sensor. The at least one physiological characteristic sensor can be any type of sensor suitable to acquire physiological characteristic data on the subject. Thus, in these embodiments, the data acquired on the subject can comprise physiological characteristic data on the subject. The at least one physiological characteristic sensor 104 may comprise any one or more of a heart rate sensor, a blood pressure sensor, a respiratory rate sensor, or any other physiological characteristic sensor, or any combination of physiological characteristic sensors, suitable to acquire physiological characteristic data on the subject.

A heart rate sensor may be any type of sensor, or any combination of sensors, suitable to acquire heart rate data from the subject. Examples of a heart rate sensor include, but are not limited to, an electrocardiogram (ECG) sensor, a photoplethysmography (PPG) sensor, a phonocardiography (PCG) sensor, a camera, or any other heart rate sensor, or any other heart rate sensor, or any combination of heart rate sensors. A blood pressure sensor may be any type of sensor, or any combination of sensors, suitable to acquire blood pressure data from the subject. Examples of a blood pressure sensor include, but are not limited to, an auscultatory sensor, an oscillometric sensor, an ultrasound sensor, photoplethysmography (PPG) sensor (for example, that measures a pulse transient time), or any other heart rate sensor, or any combination of heart rate sensors. A respiratory rate sensor may be any type of sensor, or any combination of sensors, suitable to acquire respiratory rate data from the subject. Examples of a respiratory rate sensor include, but are not limited to, a respiratory belt, an electrocardiogram (ECG) sensor, a photoplethysmography (PPG) sensor, a camera (for example, directed at the chest of the user), a motion sensor such as an accelerometer (for example, worn on the chest of the user), or any other respiratory rate sensor, or any combination of respiratory rate sensors.

Alternatively or in addition to at least one physiological characteristic sensor, the at least one sensor 104 may comprise at least one motion sensor. The at least one motion sensor can be any type of sensor suitable to acquire motion data on the subject. Thus, in these embodiments, the data acquired on the subject can comprise motion data on the subject. The at least one motion sensor may comprise any one or more of an accelerometer, a gyroscope, a magnetometer, a passive infrared sensor (PIR) sensor, a camera, or any other motion sensor, or any combination of motion sensors, suitable to acquire motion data on the subject.

Although some examples have been provided for the type of the at least one sensor 104, it will be understood that other types of sensor, or combinations of sensors, suitable to acquire data on the subject are also possible.

As illustrated in Figure 1, in some embodiments, the apparatus 100 itself can comprise a user interface 106. This user interface 106 may be the user interface that is configured to receive an input from the subject indicative of one or more symptoms of the subject. Alternatively or in addition, a user interface 106 that is configured to receive an input from the subject indicative of one or more symptoms of the subject may be external to (i.e. separate to or remote from) the apparatus 100. For example, the user interface 106 may be part of another device. In some embodiments, any one or more of a wearable device (such as a smart watch, a monitoring device, or any other wearable device) configured to be worn by the subject, a portable electronic device (for example, a smart device such as a tablet device, a smart phone, or any other portable electronic device), and any other type of device may comprise the user interface 106. In some embodiments, the apparatus 100 may comprise both the user interface 106 and at least one of the one or more sensors 104, or the user interface 106 and at least one of the one or more sensors 104 may be part of the same external device.

As mentioned earlier, the control unit 102 is configured to receive from a user interface 106 the input from the subject indicative of one or more symptoms of the subject. In addition or as an alternative to being configured to receive an input, a user interface 106 may be configured to provide information resulting from the method described herein. For example, a user interface 106 may be configured to provide (for example, render, output, or display) the one or more symptoms with the determined onset time of the one or more symptoms. The control unit 102 may be configured to control the user interface 106 to provide information resulting from the method described herein. Thus, a user interface 106 may be any user interface that enables information resulting from the method described herein to be provided (for example, rendered, output, or displayed) and, alternatively or in addition, a user interface 106 may be any user interface that enables a user of the apparatus 100 (for example, the subject themselves, or any other user of the apparatus 100) to provide a user input, interact with and/or control the apparatus 100.

For example, a user interface 106 may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a tablet, a smart phone, a smart watch, or any other smart device), or any other user interface, or combination of user interfaces. In some embodiments, the user interface 106 that is configured to provide (for example, render, output, or display) information resulting from the method described herein may be the same user interface 106 as that which enables the user to provide a user input, interact with and/or control the apparatus 100.

As illustrated in Figure 1, in some embodiments, the apparatus 100 may also comprise a memory 108. The memory 108 of the apparatus 100 can be configured to store program code that can be executed by the control unit 102 to perform the method described herein. Alternatively or in addition to the memory 108 of the apparatus 100, one or more memories 108 may be external to (i.e. separate to or remote from) the apparatus 100. For example, one or more memories 108 may be part of another device. A memory 108 can be configured to store information, data, signals and measurements that are acquired or made by the control unit 102 of the apparatus 100 or from any components, units, interfaces, sensors, memories, or devices that are external to the apparatus 100. The control unit 102 may be configured to control a memory 108 to store information, data, signals and measurements resulting from the method disclosed herein. For example, the control unit 102 may be configured to control a memory 108 to store the one or more symptoms with the determined onset time of the one or more symptoms.

According to some embodiments, as illustrated in Figure 1, the apparatus 100 may also comprise a communications interface (or circuitry) 110 for enabling the apparatus 100 to communicate with (or connect to) any components, interfaces, units, memories, sensors and devices that are internal or external to the apparatus 100. For example, the communications interface 110 may be configured to communicate with the one or more sensors 104, the user interface 106 and/or one or more memories 108. In any of the embodiments described herein, the communications interface 110 may be configured to communicate with any components, interfaces, units, memories, sensors and devices wirelessly or via a wired connection.

It will be appreciated that Figure 1 only shows the components required to illustrate this aspect of the invention and, in a practical implementation, the apparatus 100 may comprise additional components to those shown. For example, the apparatus 100 may comprise a battery or other power supply for powering the apparatus 100 or means for connecting the apparatus 100 to a mains power supply.

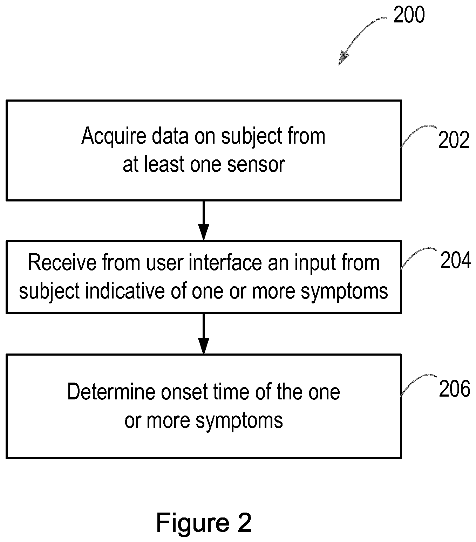

**Figure 2** illustrates a method 200 of operating the apparatus 100 comprising the control unit 102 for receiving an input indicative of one or more symptoms of a subject according to an embodiment. The illustrated method 200 can generally be performed by or under the control of the control unit 102 of the apparatus 100.

With reference to Figure 2, at block 202, data on the subject is acquired from at least one sensor 104. More specifically, the control unit 102 of the apparatus 100 acquires data on the subject from the at least one sensor 104. As previously mentioned, the at least one sensor 104 can comprise at least one physiological characteristic sensor (such as any of those mentioned earlier) and thus the data acquired on the subject can comprise physiological characteristic data on the subject. Alternatively or in addition, the at least one sensor 104 can comprise at least one motion sensor (such as any of those mentioned earlier) and thus the data acquired on the subject can comprise motion data on the subject.

At block 204 of Figure 2, an input indicative of one or more symptoms of the subject is received from a user interface 106 from the subject. More specifically, the control unit 102 of the apparatus 100 receives the input indicative of one or more symptoms of the subject from the user interface 106. The user interface 106 may receive the input indicative of one or more symptoms of the subject, for example, by the subject pressing a button on the user interface 106. The input is received at T1, whereas the subject may have felt the symptom at an earlier time T0 (which is considered to be the actual onset time of the symptom). As previously mentioned, the one or more symptoms can comprise any one or more of pain in a part of the body (such as the chest, or any other part of the body) of the subject, breathlessness of the subject, dizziness of the subject, or any other symptom, or any combination of symptoms.

At block 206 of Figure 2, an onset time of the one or more symptoms is determined by the control unit 102 based on the data acquired on the subject from the at least one sensor 104. In embodiments where the data acquired on the subject comprises physiological characteristic data on the subject, the onset time of the one or more symptoms is determined based on the physiological characteristic data acquired on the subject. In embodiments where the data acquired on the subject comprises motion data on the subject, the onset time of the one or more symptoms is determined based on the motion data acquired on the subject. In embodiments where the data acquired on the subject comprises both physiological characteristic data on the subject and motion data on the subject, the onset time of the one or more symptoms can be determined based on the physiological characteristic data acquired on the subject, or based on the motion data acquired on the subject, or based on both the physiological characteristic data and the motion data acquired on the subject.

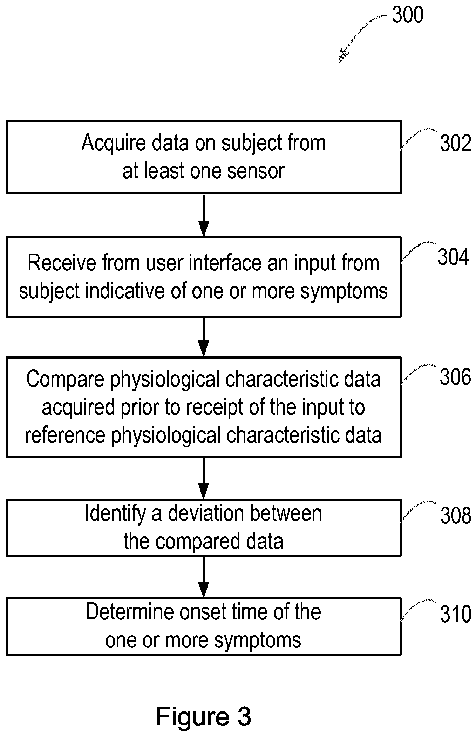

**Figure 3** illustrates a method 300 of operating the apparatus 100 comprising the control unit 102 for receiving an input indicative of one or more symptoms of a subject according to an example embodiment. The illustrated method 300 can generally be performed by or under the control of the control unit 102 of the apparatus 100.

With reference to Figure 3, at block 302, data on the subject is acquired from at least one sensor 104. More specifically, the control unit 102 of the apparatus 100 acquires data on the subject from the at least one sensor 104. In the illustrated example embodiment of Figure 3, the at least one sensor 104 comprises at least one physiological characteristic sensor (such as any of those mentioned earlier) and thus the data acquired on the subject comprises physiological characteristic data on the subject. At block 304 of Figure 3, an input indicative of one or more symptoms (such as any of those mentioned earlier) of the subject is received from a user interface 106 from the subject. More specifically, the control unit 102 of the apparatus 100 receives the input indicative of one or more symptoms of the subject from the user interface 106. The user interface 106 may receive the input indicative of one or more symptoms of the subject, for example, by the subject pressing a button on the user interface 106. The input is received at T1, whereas the subject may have felt the symptom at an earlier time T0 (which is considered to be the actual onset time).

At block 306 of Figure 3, the physiological characteristic data acquired prior to receipt of the input is compared by the control unit 102 to reference physiological characteristic data stored in a memory 108. The reference physiological characteristic data is indicative of a baseline for the physiological characteristic data. At block 308 of Figure 3, a deviation between the physiological characteristic data acquired prior to receipt of the input and the reference physiological characteristic data is identified by the control unit 102. A deviation is where a current physiological characteristic data value is above or below a reference physiological characteristic data value (for example, plus or minus a natural intra-individual variance). At block 310 of Figure 3, an onset time of the one or more symptoms is determined by the control unit 102 based on the physiological characteristic data acquired on the subject from the at least one sensor 104. In particular, the onset time of the one or more symptoms is determined as the time at which the deviation started.

In another example embodiment where the method of Figure 3 is performed, the at least one sensor 104 comprises at least one motion sensor (such as any of those mentioned earlier) as well as the at least one physiological characteristic sensor and thus the data acquired on the subject at block 302 of Figure 3 comprises both physiological characteristic data on the subject and motion data on the subject. In this example embodiment, prior to the comparison at block 306 of Figure 3, an activity in which the subject is engaging at the determined onset time is identified by the control unit 102 from the acquired motion data. Then, according to this example embodiment, the comparison that is performed at block 306 of Figure 3 comprises comparing the physiological characteristic data acquired prior to the determined onset time to the reference physiological characteristic data that is stored in the memory 108 for the same activity as the identified activity. The same activity can, for example, comprise any one or more of the same type of activity and the same intensity level of activity. Thus, in this example embodiment, the control unit 102 analyses the physiological characteristic data to determine if this physiological characteristic data is, for example, elevated or lowered in comparison to baseline reference values for the same type of activity and/or the same intensity level of activity as that performed when the one or more symptoms are reported.

For example, the subject may be jogging (which is the type of activity) at 8 km/h (which is the intensity level of the activity) when the subject experiences a symptom (for example, chest pain) and the subject provides an input indicative of this symptom at the user interface 106 at a time T1. At the time T1, the subject may in fact be standing still. The control unit 102 thus identifies the origin of the movement carried out to provide the input at the user interface 106. This allows the control unit 102 to determine the activity in which the subject was engaging when the movement was initiated at a time T0 (which is considered to be the actual onset time of the symptom). The control unit 102 then determines that at T0 the user was jogging at 8 km/h and thus compares the physiological characteristic data acquired prior to the determined onset time to reference physiological characteristic data that is stored in the memory 108 for the same activity as the identified activity, such as the same type of activity (jogging) and/or the same intensity level of activity (8 km/h). Where the control unit 102 determines a discrepancy in the compared data, meaning a current physiological characteristic data value above or below the baseline reference value (for example, plus or minus the natural intra-individual variance), the control unit 102 analyses the backward or earlier physiological characteristic data to identify the start of this deviation as the onset of the symptom.

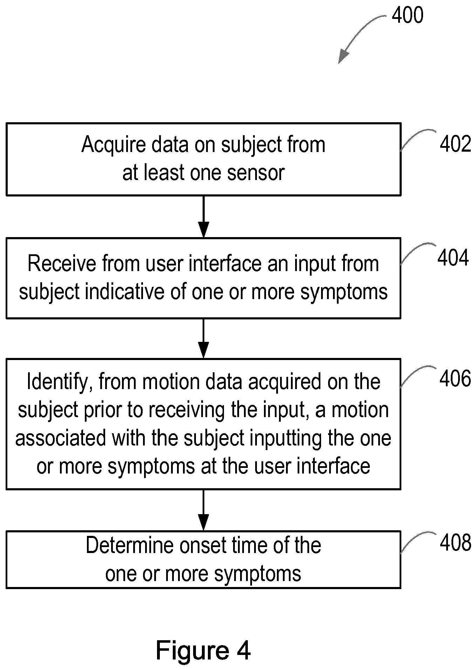

**Figure 4** illustrates a method 400 of operating the apparatus 100 comprising the control unit 102 for receiving an input indicative of one or more symptoms of a subject according to another example embodiment. The illustrated method 400 can generally be performed by or under the control of the control unit 102 of the apparatus 100.

With reference to Figure 4, at block 402, data on the subject is acquired from at least one sensor 104. More specifically, the control unit 102 of the apparatus 100 acquires data on the subject from the at least one sensor 104. In the illustrated example embodiment of Figure 4, the at least one sensor 104 comprises at least one motion sensor (such as any of those mentioned earlier) and thus the data acquired on the subject comprises motion data on the subject.

At block 404 of Figure 4, an input indicative of one or more symptoms (such as any of those mentioned earlier) of the subject is received from a user interface 106 from the subject. More specifically, the control unit 102 of the apparatus 100 receives the input indicative of one or more symptoms of the subject from the user interface 106. The user interface 106 may receive the input indicative of one or more symptoms of the subject, for example, by the subject pressing a button on the user interface 106. The input is received at T1, whereas the subject may have felt the symptom at an earlier time T0 (which is considered to be the actual onset time). The control unit 102 recognises the input as a trigger and looks into the motion data from earlier in time.

At block 406 of Figure 4, a motion (or movement, such as a gesture) associated with the subject inputting the one or more symptoms at the user interface 106 is identified by the control unit 102 from the motion data acquired on the subject prior to receiving the input. In particular, when the subject feels one or more symptoms at the time T0 (which is considered to be the onset time of the one or more symptoms), the subject initiates the motion to input or log the one or more symptoms at the user interface 106. The one or more symptoms are input or logged at the time T1. The control unit 102 identifies the moment at which the motion associated with the subject inputting the one or more symptoms at the user interface 106 is initiated or started. For example, the control unit 102 may analyse the pattern of the motion data, where the pattern of the motion data between the time T1 at which the one or more symptoms are input and the onset time T0 of the one or more symptoms is different from the pattern of the motion data prior to onset time T0, such that the control unit 102 can identify T0 as the time at which the identified motion associated with the subject inputting the one or more symptoms started.

At block 408 of Figure 4, an onset time of the one or more symptoms is determined by the control unit 102 based on the motion data acquired on the subject from the at least one sensor 104. In particular, the onset time of the one or more symptoms is determined as the time at which the identified motion associated with the subject inputting the one or more symptoms started. In other words, the time T0 at which the identified motion associated with the subject inputting the one or more symptoms started is identified as the temporal origin of the one or more symptoms. Thus, it can be determined from previously acquired motion data when a motion by the subject to input the one or more symptoms was initiated and this initiation of the motion by the subject to input the one or more symptoms can be determined as the onset time for the one or more symptoms.

In another example embodiment where the method of Figure 4 is performed, the at least one sensor 104 comprises at least one physiological characteristic sensor (such as any of those mentioned earlier) as well as the at least one motion sensor and thus the data acquired on the subject at block 402 of Figure 4 comprises both motion data on the subject and physiological characteristic data on the subject. In this example embodiment, the method further comprises the control unit 102 comparing the physiological characteristic data acquired prior to the determined onset time to reference physiological characteristic data stored in a memory 108. The reference physiological characteristic data indicative of a baseline for the physiological characteristic data.

The method then comprises the control unit 102 determining whether a deviation between the physiological characteristic data acquired prior to the determined onset time and the reference physiological characteristic data occurred. Where a deviation exceeding a threshold deviation occurred, the determined onset time of the one or more symptoms may be changed by the control unit 102 to the time at which the deviation exceeding the threshold deviation occurred. On the other hand, where no deviation occurred or a deviation less than the threshold deviation occurred, the determined onset time of the one or more symptoms may be maintained by the control unit 102 as the time at which the identified motion associated with the subject inputting the one or more symptoms started. Thus, the motion data is considered to still be valid if the physiological characteristic data is not altered, which can provide more reliable results as not all symptoms may alter the physiological characteristic data.

Thus, in this example embodiment, the control unit 102 not only analyses the motion data backward, or earlier, in time to determine the moment at which the motion (or movement such as a gesture) associated with the subject inputting the one or more symptoms at the user interface started, but also analyses the physiological characteristic data to determine if this physiological characteristic data is, for example, elevated or lowered in comparison to baseline reference values.

In any of the embodiments described herein, the method can further comprise the control unit 102 controlling a memory 108, which may be the memory of the apparatus 100 or a memory external to the apparatus 100, to store the one or more symptoms with the onset time of the one or more symptoms. Alternatively or in addition, in any of the embodiments described herein, the method can further comprise the control unit 102 outputting the one or more symptoms with the onset time of the one or more symptoms. For example, the control unit may output the one or more symptoms with the onset time of the one or more symptoms to another device and/or may control a user interface 108, which may be the user interface of the apparatus 100 or a user interface external to the apparatus 100, to output (for example, render or display) the one or more symptoms with the onset time of the one or more symptoms.

In any of the embodiments described herein, the method can further comprise the control unit 102 correlating the one or more symptoms to one or more physiological characteristics acquired at the onset time of the one or more symptoms. Alternatively or in addition, the control unit 102 may correlate the one or more symptoms to one or more behavioural parameters acquired at the onset time of the one or more symptoms, such as one or more activities (for example, walking, sitting watching television, eating, and so on) performed by the subject at the onset time of the one or more symptoms. Alternatively or in addition, the control unit 102 may correlate the one or more symptoms to one or more environmental parameters acquired at the onset time of the one or more symptoms, such as whether the subject is indoors or outdoors at the onset time of the one or more symptoms, a temperature of the environment in which the subject is present at the onset time of the one or more symptoms, a humidity of the environment in which the subject is present at the onset time of the one or more symptoms, and so on. In these embodiments, the method can also comprise the control unit 102 controlling a memory 108, which may be the memory of the apparatus 100 or a memory external to the apparatus 100, to store the correlation. Alternatively or in addition, the method according to these embodiments can also comprise the control unit 102 outputting the correlation, for example, for use in diagnosis. For example, the control unit may output the correlation to another device and/or may control a user interface 108, which may be the user interface of the apparatus 100 or a user interface external to the apparatus 100, to output (for example, render or display) the correlation.

There is therefore provided an improved apparatus and method for receiving an input indicative of one or more symptoms of a subject. The method and apparatus described herein provides a more accurate temporal localisation of the one or more symptoms, which can improve a diagnosis made for the subject using the data acquired in the manner described herein. The method and apparatus described herein can be useful in the healthcare or clinical domain, for example, where a subject is being monitored for health purposes.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (200, 300, 400) of operating an apparatus (100) comprising a control unit (102) for receiving an input indicative of one or more symptoms of a subject, the method (200) comprising:
acquiring (202, 302, 402), from at least one sensor (104), data on the subject;
receiving (204, 304, 404), from a user interface (106), an input from the subject indicative of one or more symptoms of the subject; and
determining (206, 310, 408) an onset time of the one or more symptoms based on the data acquired on the subject from the at least one sensor (104).

2. A method (200, 300, 400) as claimed in claim 1, wherein the at least one sensor (104) comprises at least one physiological characteristic sensor and the data acquired on the subject comprises physiological characteristic data on the subject.

3. A method (300) as claimed in claim 2, wherein determining (310) an onset time:
comparing (306) the physiological characteristic data acquired prior to receipt of the input to reference physiological characteristic data stored in a memory, the reference physiological characteristic data indicative of a baseline for the physiological characteristic data;
identifying (308) a deviation between the physiological characteristic data acquired prior to receipt of the input and the reference physiological characteristic data; and
determining (310) the onset time of the one or more symptoms as the time at which the deviation started.

4. A method (300) as claimed in claim 3, wherein the at least one sensor (104) comprises at least one motion sensor, the data acquired on the subject comprises motion data on the subject, and the method further comprises:
prior to comparing, identifying from the acquired motion data an activity in which the subject is engaging at the determined onset time; and
wherein comparing (306) comprises:
comparing the physiological characteristic data acquired prior to the determined onset time to the reference physiological characteristic data that is stored in the memory for the same activity as the identified activity.

5. A method (300) as claimed in claim 4, wherein the same activity comprises any one or more of the same type of activity and the same intensity level of activity.

6. A method (200, 300, 400) as claimed in any one of claims 1 or 2, wherein the at least one sensor (104) comprises at least one motion sensor and the data acquired on the subject comprises motion data on the subject.

7. A method (400) as claimed in claim 6, wherein determining (408) an onset time comprises:
identifying (406), from the motion data acquired on the subject prior to receiving the input, a motion associated with the subject inputting the one or more symptoms at the user interface; and
determining (408) the onset time of the one or more symptoms as the time at which the identified motion associated with the subject inputting the one or more symptoms started.

8. A method (400) as claimed in claim 7, when claim 6 is dependent on claim 2, the method further comprising:
comparing the physiological characteristic data acquired prior to the determined onset time to reference physiological characteristic data stored in a memory, the reference physiological characteristic data indicative of a baseline for the physiological characteristic data;
determining whether a deviation between the physiological characteristic data acquired prior to the determined onset time and the reference physiological characteristic data occurred; and
where a deviation exceeding a threshold deviation occurred, changing the determined onset time of the one or more symptoms to the time at which the deviation exceeding the threshold deviation occurred; and
where no deviation occurred or a deviation less than the threshold deviation occurred, maintaining the determined onset time of the one or more symptoms as the time at which the identified motion associated with the subject inputting the one or more symptoms started.

9. A method (200, 300, 400) as claimed in any one of the preceding claims, the method further comprising any one or more of:
controlling a memory (108) to store the one or more symptoms with the onset time of the one or more symptoms; and
outputting the one or more symptoms with the onset time of the one or more symptoms.

10. A method (200, 300, 400) as claimed in any one of the preceding claims, the method further comprising:
correlating the one or more symptoms to one or more physiological characteristics acquired at the onset time of the one or more symptoms, one or more behavioural parameters acquired at the onset time of the one or more symptoms, and/or one or more environmental parameters acquired at the onset time of the one or more symptoms.

11. A method (200, 300, 400) as claimed in claim 10, the method further comprising any one or more of:
controlling a memory (108) to store the correlation; and
outputting the correlation for use in diagnosis.

12. A method (200, 300, 400) as claimed in any one of the preceding claims, wherein:
the one or more symptoms comprise any one or more of:
pain in a part of the body of the subject;
breathlessness of the subject; and
dizziness of the subject; and/or
the physiological characteristic data comprises any one or more of:
a heart rate data of the subject;
a blood pressure data of the subject; and
a respiratory rate data of the subject.

13. A computer program product comprising a computer readable medium, the computer readable medium having computer readable code embodied therein, the computer readable code being configured such that, on execution by a suitable computer or processor, the computer or processor is caused to perform the method of any one of claims 1 to 12.

14. An apparatus (100) for receiving an input indicative of one or more symptoms of a subject, the apparatus (100) comprising:
a control unit (102) configured to:
acquire, from at least one sensor (104), data on the subject;
receive, from a user interface (106), an input from the subject indicative of one or more symptoms of the subject; and
determine an onset time of the one or more symptoms based on the data acquired on the subject from the at least one sensor (104).

15. An apparatus (100) as claimed in claim 14, wherein the apparatus (100) comprises one or more of the at least one sensors (104) and/or the user interface (106).
